# FASCICULE DE BREVET EUROPEEN

(11) **EP 1 093 797 B1**
(45) Date de publication et mention de la délivrance du brevet: **20.02.2002**
(21) Numéro de dépôt: 00402809.8
(22) Date de dépôt: 11.10.2000
(51) Int. Cl.: A61K 7/42

(54) **Procédé de photostabilisation de filtres solaires derivés du dibenzoylmethane par un filtre organique insoluble**
Verfahren zur Photostabilisierung von Sonnenschutzmitteln aus Dibenzoylmethanderivaten mit einem unlöslichen organischen Filter
Process for the photostabilisation of sunscreens derived from dibenzoylmethane by an insoluble organic filter

(30) Priorité: 22.10.1999 FR 9913222
(43) Date de publication de la demande: 25.04.2001
(73) Titulaire: L'OREAL, 75008 Paris (FR)
(72) Inventeur: Candau, Didier, 91570 Bievres (FR); Pisson, Anne-Marie, 91800 Boussy-Saint-Antoine (FR)
(74) Mandataire: Miszputen, Laurent

(56) Documents cités:
- EP-A- 0 824 909
- EP-A- 0 848 946
- EP-A- 0 893 119
- FR-A- 2 768 053
- US-A- 5 733 532

## Description

L'invention se rapporte à un procédé pour améliorer la stabilité d'au moins un dérivé du dibenzoylméthane vis-à-vis du rayonnement UV consistant à associer au dit dérivé de dibenzoylméthane une quantité efficace d'au moins un filtre UV organique insoluble sous forme micronisée dont la taille moyenne des particules varie de 0,01 à 2 µm.

L'invention se rapporte également à l'utilisation d'un filtre UV organique insoluble sous forme micronisée dont la taille moyenne des particules varie de 0,01 à 2 µm dans la préparation d'une composition cosmétique ou dermatologique contenant au moins un dérivé du dibenzoylméthane, pour améliorer la stabilité dudit dérivé du dibenzoylméthane vis-à-vis du rayonnement UV.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoylméthane est par ailleurs actuellement proposé à la vente sous la dénomination commerciale de "PARSOL 1789" par la Société HOFFMANN LAROCHE.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

Or, la Demanderesse vient maintenant de découvrir, de façon surprenante, qu'en associant aux dérivés du dibenzoylméthane mentionnés ci-dessus une quantité efficace d'un filtre UV organique insoluble sous forme micronisée , il était possible d'améliorer de manière substantielle et remarquable, la stabilité photochimique (ou photostabilité) de ces mêmes dérivés du dibenzoylméthane.

Ainsi, conformément à l'un des objets de la présente invention, il est maintenant proposé un nouveau procédé de stabilisation des dérivés du dibenzoylméthane vis-à-vis du rayonnement UV (longueurs d'ondes comprises entre 280 nm et 400 nm environ), en particulier du rayonnement solaire, caractérisé par le fait qu'il consiste à associer aux dits dérivés du dibenzoylméthane une quantité efficace d'au moins un filtre UV organique insoluble sous forme micronisée.

Par filtre UV organique insoluble, on entend, au sens de la présente invention, des filtres UV organiques dont la solubilité dans l'eau à 25°C est inférieure à 0,1 % en poids, dont la solubilité à 25°C dans l'huile de paraffine est inférieure à 1% en poids.

Par quantité efficace de filtre UV organique insoluble conforme à l'invention, on entend une quantité suffisante pour obtenir une amélioration notable et significative de la photostabilité du ou des dérivés du dibenzoylméthane de la composition cosmétique photoprotectrice. Cette quantité minimale en agent photostabilisant à mettre en oeuvre. qui peut varier selon la nature du support cosmétiquement acceptable retenu pour la composition, peut être déterminée sans aucune difficulté au moyen d'un test classique de mesure de photostabilité, tel que celui donné dans les exemples ci-après.

La présente invention a également enfin pour objet l'utilisation d'un filtre UV organique insoluble dans la préparation d'une composition cosmétique ou dermatologique comprenant au moins un dérivé du dibenzoylméthane dans le but de d'améliorer la stabilité vis-à-vis des rayons UV ledit dérivé du dibenzoylméthane contenu.

D'autres caractéristiques, aspects et avantages de la présente invention apparaîtront à la lecture de la description détaillée qui va suivre.

Les filtres organiques insolubles selon l'invention se présentent sous forme insoluble micronisée; la taille moyenne des particules varie de 0,01 à 2 µm et plus préférentiellement de 0,02 à 1,5 µm et plus particulièrement de 0,03 à 1,0 µm.

Ils peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent être en particulier obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893 119. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure CₙH ₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C₁-C₁₂ d'un composé de structure CₙH₂ₙ₊₁ O(C₆H₁₀O₅)ₓH et plus précisément un ester obtenu par réaction d'un acide carboxylique en C1-C12 tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre insoluble dans sa forme micronisée.

Comme indiqué précédemment, les dérivés du dibenzoylméthane destinés à être photostabilisés dans le cadre de la présente invention sont des produits déjà bien connus en soi et décrits notamment dans les documents FR-A-2 326 405, FR-A-2 440 933 et EP-A-0 114 607 précités.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

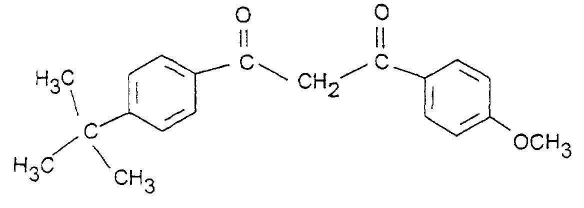

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

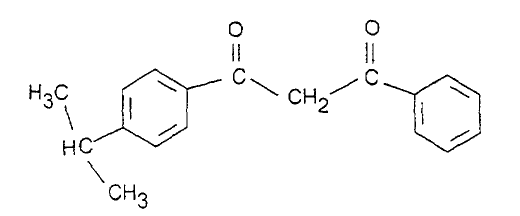

Le ou les dérivés du dibenzoylméthane peuvent être présents dans les compositions conformes à l'invention, ou dans les compositions destinées à être stabilisées conformément au procédé de l'invention, à des teneurs qui sont généralement comprises entre 0,01 % et 10 % en poids, et de préférence à des teneurs comprises entre 0,1 % et 6 % en poids, par rapport au poids total de la composition.

Les filtres UV organiques insolubles conformes à l'invention peuvent être choisis notamment parmi les filtres UV organiques du type oxanilide, du type triazine, du type triazole, du type amide vinylique, du type cinnamide.

Parmi les filtres UV du type oxanilide conformes à l'invention, on peut citer ceux répondant à la structure : 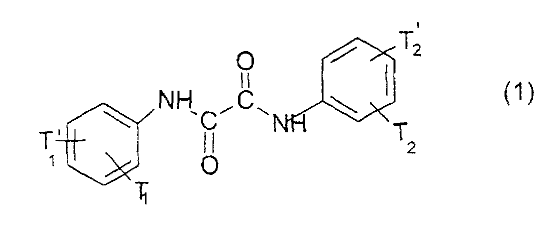 dans laquelle T₁, T'₁, T₂ et T'₂ désignent, identiques et différents, un radical alkyle en C₁-C₈ ou un radical alcoxy en C₁-C₈. Ces composés sont décrits dans la demande de brevet WO95/22959.

A titre d'exemples , on peut citer les produits commerciaux TINUVIN 315 et TINUVIN 312 vendus par la Société CIBA-GEIGY et respectivement de structure :

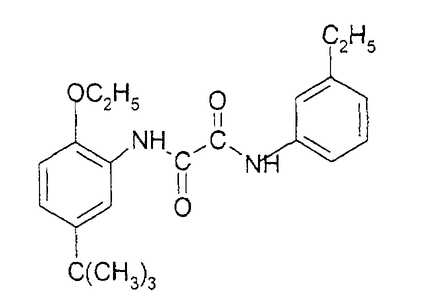

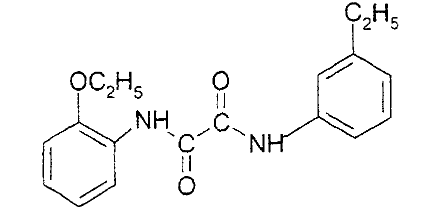

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243.

Parmi ces filtres UV du type triazine, on citera plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Parmi les filtres UV du type triazine conformes à l'invention, on peut également mentionner ceux répondant à la formule (2) suivante : 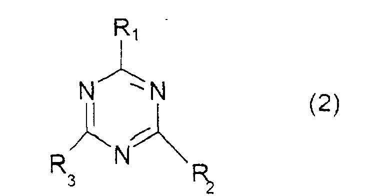 dans laquelle R₁, R₂, R₃,, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR₄, avec R₄ soit C₁-C₁₈alkyle soit cinnamyle, et n vaut 0 ou 1.

Ces composés sont décrits dans WO 97/03642, GB 2286774, EP-743309, WO 98/22447, GB 2319523.

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922.

Parmi ces composés, on peut citer plus particulièrement :
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Parmi les filtres UV organiques du type triazole conformes à l'invention, on peut citer ceux de formule suivante (3) tels que décrits dans la demande WO95/22959: 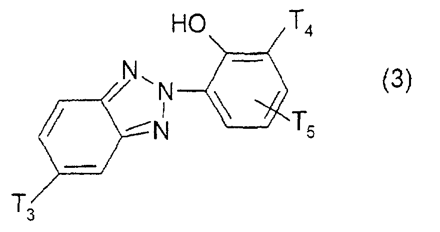 dans laquelle T₃ désigne un atome d'hydrogène ou un radical alkyle en C₁-C₁₈; T₄ et T₅, identiques ou différents, désignent un radical alkyle en C₁-C₁₈ éventuellement substitué par un phényle.

A titre d'exemple de composés de formule (3), on peut citer les produits commerciaux TINUVIN 328, 320, 234 et 350 de la Société CIBA-GEIGY de structure suivante:

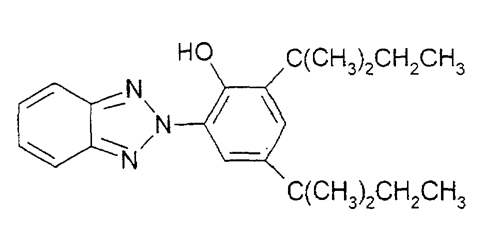

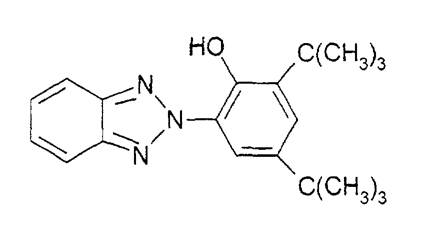

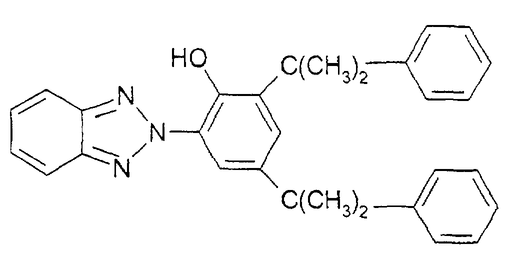

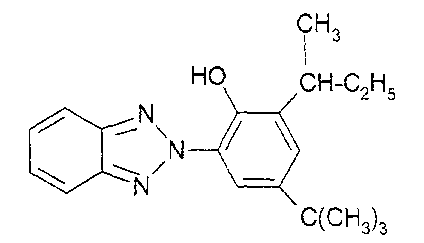

Parmi les filtres UV organiques du type triazole conformes à l'invention, on peut citer les composés tels que décrits dans les brevets US 5 687 521, US 5 687 521, US 5 373 037, US 5 362 881 et en particulier le [2 ,4'-dihydroxy-3-(2H-benzotriazol-2-yl)-5-(1,13,3-tétraméthylbutyl)-2'-n-octoxy-5'-benzoyl] diphénylméthane vendu sous le nom MIXXIM PB30 par la société FAIRMOUNT CHEMICAL de structure :

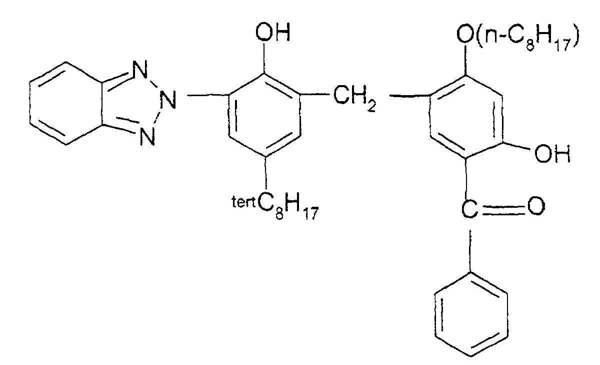

Parmi les filtres UV organiques du type benzotriazole conformes à l'invention, on peut citer les dérivés de méthylène bis-(hydroxyphényl benzotriazole) de structure suivante : 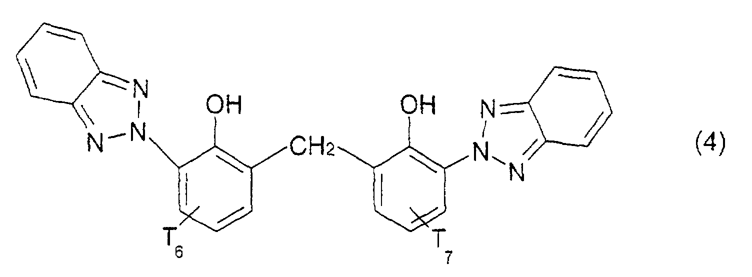 dans laquelle les radicaux T₆ et T₇, identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle. Ces composés sont connus en soi et décrits dans les demandes US 5237 071, US 5 166 355, GB-A-2 303 549, DE 197 26 184 et EP-A-893 119.

Dans la formule (4) définie ci-dessus : les groupes alkyle en C₁-C₁₈ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle; les groupes cycloalkyle en C₅-C₁₂ sont par exemple cyclopentyle, cyclohexyle, cyclooctyle ; les groupes aryle sont par exemple phényle, benzyle.

Parmi les composés de formule (4), on préfère plus particulièrement ceux de structure suivante :

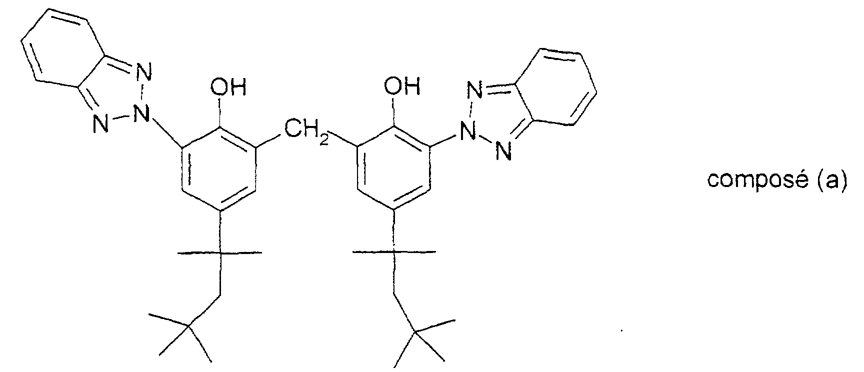

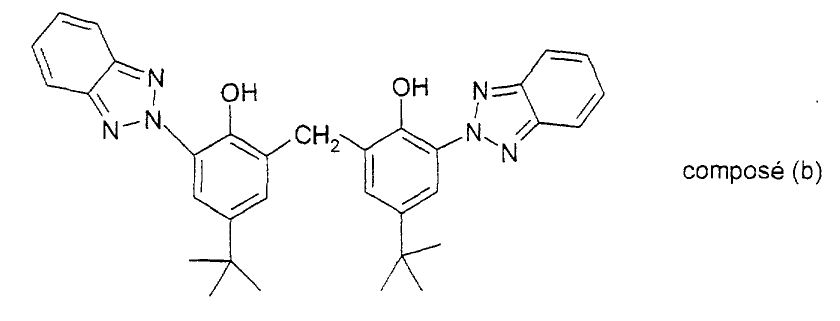

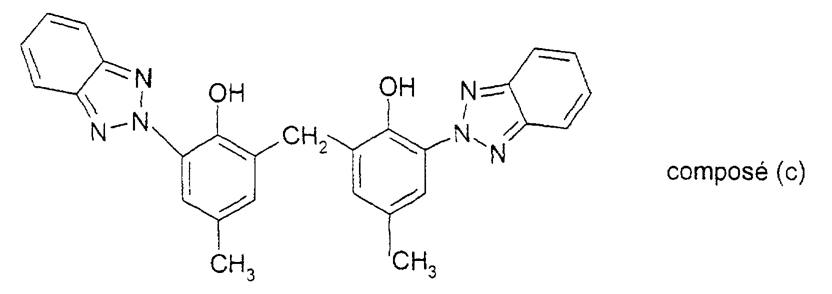

Le composé (a) de nomenclature 2,2'-méthyiène-bis-[6-(2H-benzotriazol-2-yl)-4-(1,1,3,3-tetraméthylbutyl)phénol] est vendu sous le nom MIXXIM BB/100 par le société FAIRMOUNT CHEMICAL et sous forme micronisée sous le nom TINOSORB M par la société CIBA GEIGY.

Le composé (c) de nomenclature 2,2'-méthylène-bis-[6-(2H-benzotriazol-2-yl)-4-(methyl)phénol] est vendu sous le nom MIXXIM BB/200 par le société FAIRMOUNT CHEMICAL.

Parmi les filtres organiques insolubles du type amide vinylique, on peut citer par exemple les composés de formules suivante qui sont décrits dans la demande WO95/22959 :

T₈-(Y)r-C(=O)-C(T₉)=C(T₁₀)-N(T₁₁)(T₁₂) (5)

dans laquelle T₈ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₃ où T₁₃ est un alkyle en C₁-C₁₈ ; T₉, T₁₀, T₁₁ et T₁₂ identiques ou différents désignent un radical alkyle en C₁-C₁₈, de préférence en C₁-C₅ ou un atome d'hydrogène ; Y est N ou O et r vaut 0 ou 1.

Parmi ces composés, on citera plus particulièrement :
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

Parmi les filtres organiques du type cinnamamide conformes à l'invention, on peut citer également les composés tels que décrits dans la demande WO95/22959 et répondant à la structure suivante : 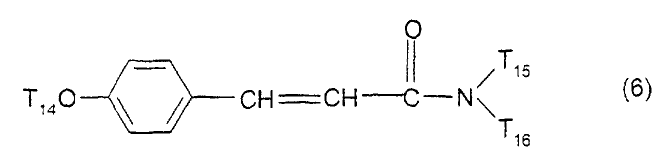 dans laquelle OT₁₄ est un radical hydroxy ou alcoxy en C₁-C₄, de préférence méthoxy ou éthoxy ; T₁₅ est hydrogène, alkyle en C₁-C₄, de préférence méthyle ou éthyle ; T₁₆ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₁₇ où T₁₇ est un alkyle en C₁-C₁₈ et plus préférentiellement T₁₇ est un groupe phényle, 4-méthoxyphényle ou phénylaminocarbonyle.

On peut également citer les dimères cinnamamides tels que ceux décrits dans le brevet US 5888481 comme par exemple le composé de structure :

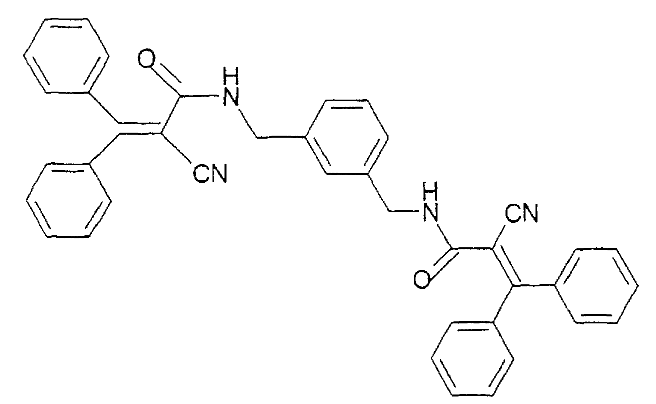

Une autre famille particulière de filtres organiques insolubles conformes à l'invention sont les sels de métaux polyvalents (par exemple Ca ²⁺, Zn ²⁺, Mg ²⁺, Ba ²⁺, Al ³⁺ ou Zr ⁴⁺) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893 119 ; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres organiques insolubles micronisés selon l'invention sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Un autre objet de l'invention consiste en des compositions cosmétiques ou dermatologiques caractérisées par le fait qu'elle comprennent au moins une émulsion telle que définie précédemment.

Les compositions cosmétiques antisolaires selon l'invention peuvent bien entendu contenir un ou plusieurs filtres organiques complémentaires actifs dans l'UVA et/ou l'UVB (absorbeurs), liposolubles ou hydrosolubles. Ces filtres complémentaires peuvent être notamment choisis parmi les dérivés salicyliques, les dérivés du camphre ; les dérivés de triazine tels que ceux décrits dans les demandes de brevet US 4367390, EP863145, EP517104, EP570838, EP796851, EP775698, EP878469 et EP 933376 ; les dérivés de la benzophénone ; les dérivés de β,β'-diphénylacrylate, les dérivés de benzimidazole ;; les dérivés bis-benzoazolyle tels que décrits dans les brevets EP-A-0669323 et US 2,463,264; les dérivés de l'acide p-aminobenzoïque ; les polymères filtres et silicones filtres tels que ceux décrits notamment dans la demande WO-93/04665.

Comme exemples de filtres solaires complémentaires actifs dans l'UV-A et/ou l'UV-B, liposolubles ou hydrosolubles, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
l'acide benzène 1,4-di(3-méthylidène-10-camphosulfonique) et ses sels,
l'acide urocanique,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide,
l'acide 1,4-bisbenzimidazolyl-phénylen-3,3',5,5'-tétrasulfonique et ses sels.
les polyorganosiloxanes à fonction benzalmalonate
les polyorganosiloxanes à fonction benzotriazole tel que le Drometrizole Trisiloxane.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les polymères, les charges, les séquestrants, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier le niveau de photoprotection, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de poudre, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention vont maintenant être donnés. On a réalisé les compositions A, B, C et D suivantes (les quantités sont exprimées en poids % par rapport au poids total de la composition) ;

| **SUPPORT COMMUN** | **% en poids** |
|---|---|
| Mélange d'alcool cétylstéarylique et d'alcool cétylstéarylique oxyéthyléné (33 OE) 80/20 (SINNOWAX AO-HENKEL) | 7 |
| Mélange de mono et distéarate de glycérol (CERASYNT SD-V ISP) | 2 |
| Alcool cétylique | 1,6 |
| Polydiméthylsiloxane (DOW CORNING 200 FLUID -DOW CORNING) | 1,6 |
| Benzoate d'alcools en C₁₂/C₁₅ (WITCONOL TN -WITCO) | 16 |
| 4-tert-butyl 4'-méthoxy-dibenzoylméthane vendu sous le nom Parsol 1789 | X%MA |
| Glycérine | 16 |
| 2,2'-méthylène-bis-[6-(2H-benzotriazol-2-yl)-4-(1,1,3,3-tetraméthylbutyl)phénol] sous forme micronisée vendue sous le nom TINOSORB M par CIBA GEIGY Taille moyenne de particule 0,15-0,2 µm | Y% MA |
| Conservateurs | qs |
| EDTA | 0,1 |
| Eau déminéralisée qsp | 100 |

| **Exemples de composition** | **2,2'-méthylène-bis-[6-(2H-benzotriazol-2-yl)-4-(1,1,3,3-tetraméthylbutyl) phénol] sous forme micronisée** | **4-tert-butyl-4'-méthoxy-dibenzoylméthane** |
|---|---|---|
| | **Y%** | **X%** |
| **A** | 1,0 | 0,5 |
| **B** | 10,0 | 0,5 |
| **C** | 10,0 | 2,0 |
| **D** | 1,0 | 2,0 |

Pour chacune de ces compositions, on a déterminé le pourcentage de 4-tert-butyl-4'-méthoxydibenzoylméthane résiduel et le pourcentage de 2,2'-méthylène-bis-[6-(2H-benzotriazol-2-yl)-4-(1,1,3,3-tetraméthylbutyl)phénol] résiduel après irradiation par des UV selon le protocole suivant :

Pour chaque formule on a préparé 3 échantillons tests et 3 échantillons témoins. On a déposé à la spatule 2 mg/cm² de formule sur des plaques de polyméthacrylate de méthyl. Les plaques tests ont été exposées 1h39mn au Suntest HERAEUS muni d'une lampe XENON ayant comme Flux UVA : 3,02.10⁻³ W/cm² et Flux UVB : 2,05.10⁻⁴ W/cm² et les plaques témoins sont conservées pendant le même temps et à la même température (35-40°C) à l'obscurité. A l'issue de ce temps, on a procédé à l'extraction des filtres en immergeant chaque plaque dans 50g de tétrahydrofurane, et en les passant aux ultrasons pendant 15mn pour assurer une bonne extraction. Les solutions obtenues sont analysées par chromatographie en phase liquide haute performance. Pour chaque formule testée, le taux de dibenzoylméthane résiduel après exposition est donné par le rapport de sa concentration dans l'échantillon exposé à sa concentration dans l'échantillon non exposé. Les résultats obtenus sont rassemblés dans le tableau ci-dessous :

| **Composition** | **Irradiation 1 heure UVA solaire Fraction résiduelle** | |
|---|---|---|
| **Filtre UV** | 2,2'-méthylène-bis-[6-(2H-benzotriazol-2-yl) -4-(1,1,3,3-tetraméthylbutyl)phénol] sous forme micronisée | 4-tertio-butyl-4'-méthoxydibenzoylméthane |
| **A** | (102 ± 2) % | (53 ± 2)% |
| **B** | (100 ± 2)% | (70 ± 2)% |
| **C** | (99 ± 3) % | (50 ± 3) % |
| **D** | (98 ± 1) % | (41 ± 4)% |

On observe que le filtre UV organique insoluble permet d'améliorer la photostabilité du dérivé de dibenzoylméthane vis à vis des rayonnements UV.

## Revendications

1. Procédé pour améliorer la stabilité d'au moins un dérivé du dibenzoylméthane vis-à-vis du rayonnement UV, **caractérisé par le fait qu'**il consiste à associer audit dérivé du dibenzoylméthane une quantité efficace d'au moins un filtre UV organique insoluble sous forme insoluble micronisée avec une taille moyenne de particule allant de 0,01 à 2µm, ledit filtre UV organique insoluble ayant une solubilité à 25°C dans l'eau inférieure à 0,1% en poids et une solubilité à 25°C dans l'huile de paraffine inférieure à 1% en poids.

2. Procédé selon la revendication 1, où la taille moyenne de particule varie de 0,02 à 1,5 µm.

3. Procédé selon la revendication 2, où la taille moyenne de particule varie de 0,03 à 1,0 µm.

4. Procédé selon l'une quelconque des revendications 1 à 4, **caractérisé par le fait que** le ou les filtres UV insolubles sous forme micronisée sont susceptibles d'être obtenus par un procédé de broyage du filtre organique insoluble sous forme de particules de taille grossière en présence d'un tensio-actif.

5. Procédé selon la revendication 4, où le tensio-actif est choisi parmi les alkylpolyglucosides de structure CₙH₂ₙ₊₁O(C₆H₁₀O₅)ₓH dans laquelle n est un entier de 8 à 16 et x est le degré moyen de polymérisation de l'unité (C₆H₁₀O₅) et varie de 1,4 à 1,6.

6. Procédé selon l'une quelconque des revendications 4 à 5, **caractérisé par le fait que** le tensio-actif est utilisé à une concentration allant de 1 à 50% en poids par rapport au filtre UV organique insoluble dans sa forme micronisée.

7. Procédé selon l'une quelconque des revendications 1 à 6, **caractérisé par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoytméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert,-butyl-4'-méthoxydibenzoylméthane.

8. Procédé selon la revendication 7, **caractérisé par le fait que** le dérivé de dibenzoylméthane est le 4-(ter.-butyl) 4'-méthoxy dibenzoylméthane.

9. Procédé selon la revendication 7, **caractérisé par le fait que** le dérivé de dibenzoylméthane est le 4-isopropyl-dibenzoylméthane.

10. Procédé selon l'une quelconque des revendications 1 à 4, où le ou les filtres UV organiques insolubles sous forme micronisée sont choisis parmi les filtres UV organiques du type oxanilide, du type triazine, du type triazole, du type amide vinylique ou du type cinnamide.

11. Procédé selon la revendication 10, où les filtres UV du type oxanilide est de formule 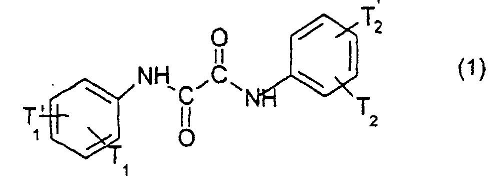 dans laquelle T₁, T'₁, T₂ et T'₂ désignent, identiques et différents, un radical alkyle en C₁-C₈ ou un radical alcoxy en C₁-C₈.

12. Procédé selon la revendication 10, où les filtres UV du type triazine sont choisis parmi les dérivés insolubles de s-triazine portant des groupements benzalmalonates et/ou phénylcyanoacrylates.

13. Procédé selon la revendication 12, où les filtres UV du type triazine sont choisis parmi les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

14. Procédé selon la revendication 10, où les filtres UV insolubles du type triazine sont choisis parmi 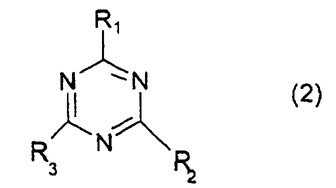 dans laquelle R₁, R₂, R₃,, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR₄, avec R₄ soit C₁-C₁₈alkyle soit cinnamyle, et n vaut 0 ou 1.

15. Procédé selon la revendication 10, où les filtres UV du type triazine sont choisis parmi les dérivés insolubles de s-triazine portant des groupements benzotriazoles et/ou benzothiazoles.

16. Procédé selon la revendication 15, où les filtres UV du type triazine sont choisis parmi
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

17. Procédé selon la revendication 10, où les filtres UV organiques du type triazole répondent à la formule (3) suivante : 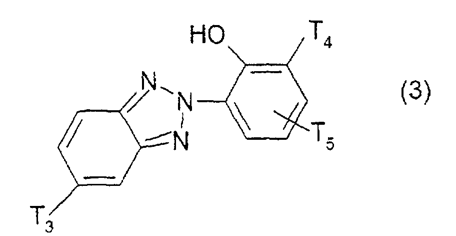 dans laquelle T₃ désigne un atome d'hydrogène ou un radical alkyle en C₁-C₁₈; T₄ et T₅, identiques ou différents, désignent un radical alkyle en C₁-C₁₈ éventuellement substitué par un phényle.

18. Procédé selon la revendication 17, où le composé de formule (3) est choisi parmi les composés suivants :

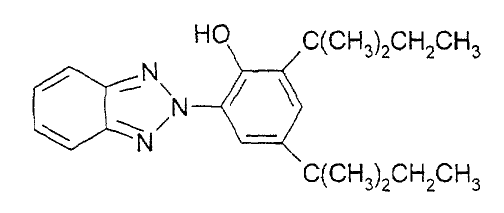

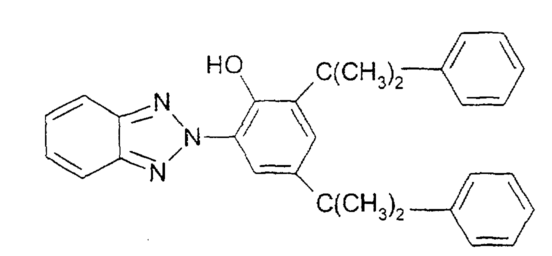

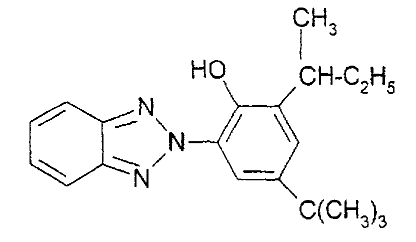

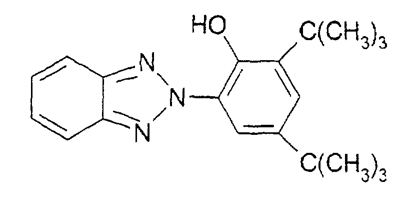

19. Procédé selon la revendication 10, où le filtre UV insoluble est le [2 ,4'-dihydroxy-3-(2H-benzotriazol-2-yl)-5-(1,1,3,3-tétraméthylbutyl)-2'-n-octoxy-5'-benzoyl] diphénylméthane de structure :

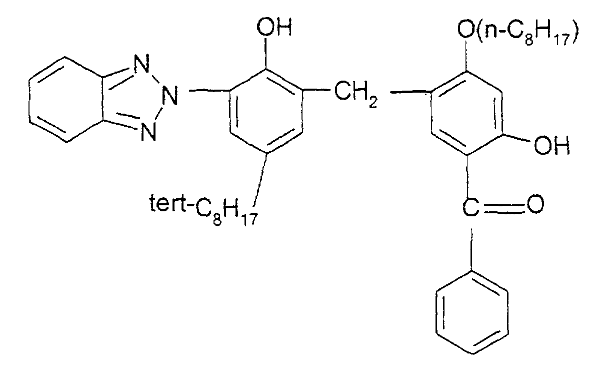

20. Procédé selon la revendication 10, où les filtres UV organiques du type triazole sont choisis parmi les dérivés de méthylène bis-(hydroxyphényl benzotriazole) de structure suivante : 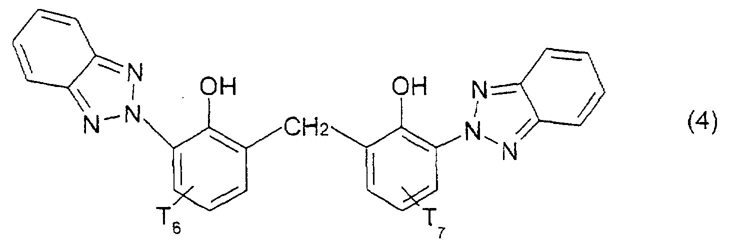 dans laquelle les radicaux T₆ et T₇, identiques ou différents, désignent un radical alkyle en C₁-C₁₈ pouvant être substitué par un ou plusieurs radicaux choisis parmi alkyle en C₁C₄, cycloalkyle en C₅-C₁₂ ou un reste aryle.

21. Procédé selon la revendication 20, où le composé de formule (4) est choisi dans le groupe constitué par les composés de structure suivante :

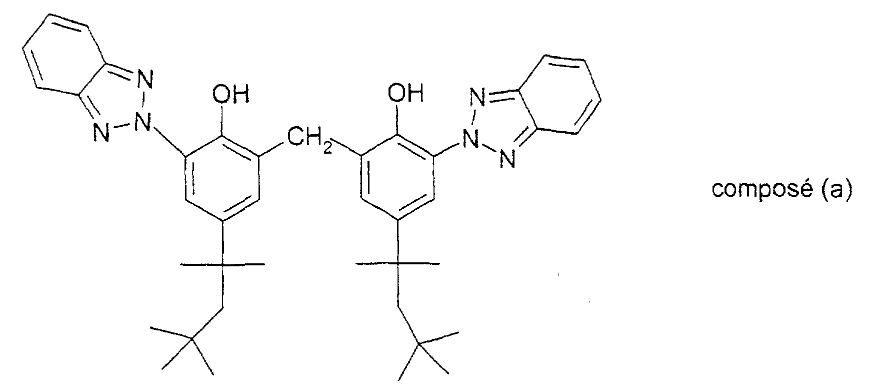

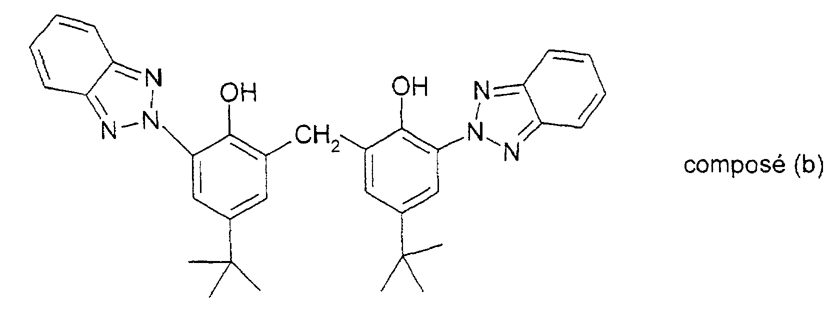

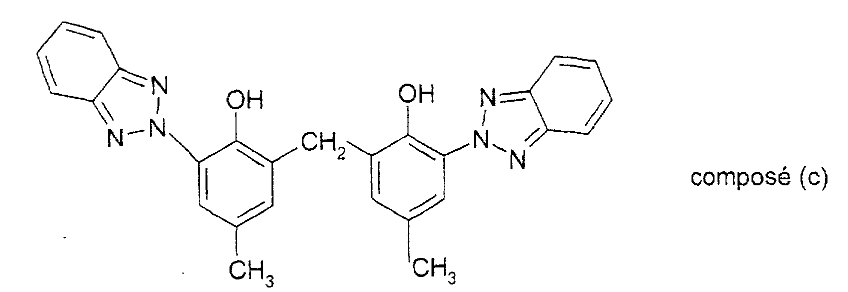

22. Procédé selon la revendication 10, où les filtres organiques du type amide vinylique, répondent à la formule suivante :
T₈-(Y)r-C(=O)-C(T₉)=C(T₁₀)-N(T₁₁)(T₁₂) (5)
dans laquelle T₈ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₃ où T₁₃ est un alkyle en C₁-C₁₈; T₉, T₁₀, T₁₁ et T₁₂ identiques ou différents désignent un radical alkyle en C₁-C₁₈, de préférence en C₁-C₅ ou un atome d'hydrogène ; Y est N ou O et r vaut 0 ou 1.

23. Procédé selon la revendication 22, où les composés de formule (5) sont choisis parmi:
- la 4-octylamino-3-pentèn-2-one ;
- l'éthyl-3-octylamino-2-buténoate;
- la 3-octylamino-1-phényl-2-butèn-1-one
- la 3-dodecylamino-1-phenyl-2-buten-1-one.

24. Procédé selon la revendication 10, où les filtres organiques du type cinnamamide répond à la formule suivante ; 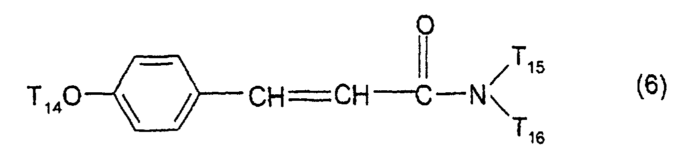 dans laquelle OT₁₄ est un radical hydroxy ou alcoxy en C₁-C₄, de préférence méthoxy ou éthoxy ; T₁₅ est hydrogène, alkyle en C₁-C₄, de préférence méthyle ou éthyle ; T₁₆ est un groupe -(CONH)s-phényle où s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₁₇ où T₁₇ est un alkyle en C₁-C₁₈ et plus préférentiellement T₁₇ est un groupe phényle, 4-méthoxyphényle ou phénylaminocarbonyle.

25. Procédé selon la revendication 10, où le filtre UV insoluble est un dimère cinnamamide.

26. Procédé selon la revendication 25, où le filtre UV insoluble est le composé de structure :

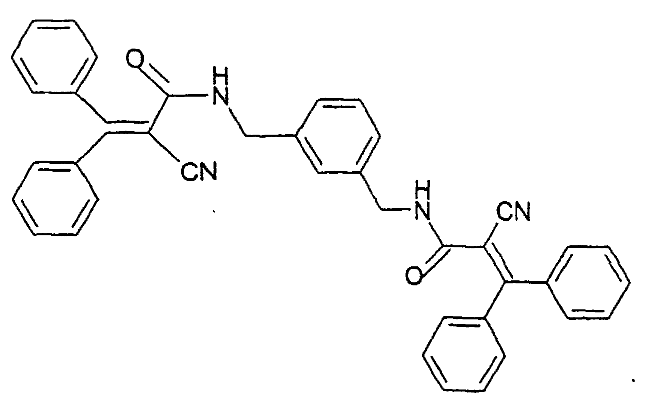

27. Procédé selon l'une quelconque des revendications 1 à 9, où les filtres UV insolubles sont des sels de métaux polyvalents de filtres UV organiques sulfoniques ou carboxyliques.

28. Procédé selon la revendication 27, où les filtres UV insolubles sont choisis parmi les sels de métaux polyvalents de dérivés sulfonés de benzylidène camphre ; les sels de métaux polyvalents de dérivés sulfonés de benzimidazole ; les sels de métaux polyvalents de dérivés d'acide cinnamique.

29. Procédé selon l'une quelconque des revendications 1 à 9, où les filtres UV insolubles sont des complexes de métaux polyvalents ou d'ammonium ou d'ammonium substitué de filtres organiques UV-A et/ou UV-B.

30. Utilisation d'un filtre UV organique insoluble sous forme micronisée tel que défini selon l'une quelconque des revendications 1 à 29, dans la préparation d'une composition cosmétique ou dermatologique contenant au moins un dérivé du dibenzoylméthane, pour améliorer la stabilité dudit dérivé du dibenzoylméthane vis-à-vis du rayonnement UV.

## Patentansprüche

1. Verfahren zur Verbesserung der Stabilität mindestens eines Dibenzoylmethanderivats gegenüber UV-Strahlung, **dadurch gekennzeichnet, daß** es darin besteht, das Dibenzoylmethanderivat mit einer wirksamen Menge eines unlöslichen organischen Filters in mikronisierter Form mit einer mittleren Größe der Partikel im Bereich von 0,01 bis 2 µm zu kombinieren, wobei das unlösliche organische Filter bei 25 °C in Wasser eine Löslichkeit unter 0,1 Gew.-% und bei 25 °C in Paraffinöl eine Löslichkeit unter 1 Gew.-% aufweist.

2. Verfahren nach Anspruch 1, wobei die mittlere Größe der Partikel im Bereich von 0,02 bis 1,5 µm liegt.

3. Verfahren nach Anspruch 2, wobei die mittlere Größe der Partikel im Bereich von 0,03 bis 1,0 µm liegt.

4. Verfahren nach einem der Ansprüche 1 bis 4, **dadurch gekennzeichnet, daß** das oder die unlöslichen UV-Filter in mikronisierter Form nach einem Zerkleinerungsverfahren erhalten werden können, bei dem das in Form von groben Partikeln vorliegende unlösliche organische Filter in Gegenwart eines grenzflächenaktiven Stoffes zerkleinert wird.

5. Verfahren nach Anspruch 4, wobei der grenzflächenaktive Stoff unter den Alkylpolyglucosiden der Struktur CₙH₂ₙ₊₁O(C₆H₁₀O₅)ₓH ausgewählt wird, wobei n eine ganze Zahl im Bereich von 8 bis 16 bedeutet und x der mittlere Polymerisationsgrad der Einheit (C₆H₁₀O₅) ist und im Bereich von 1,4 bis 1,6 liegt.

6. Verfahren nach einem der Ansprüche 4 bis 5, **dadurch gekennzeichnet, daß** der grenzflächenaktive Stoff in einer Konzentration von 1 bis 50 Gew.-%, bezogen auf das unlösliche organische Filter in mikronisierter Form, verwendet wird.

7. Verfahren nach einem der Ansprüche 1 bis 6, **dadurch gekennzeichnet, daß** das Dibenzoylmethanderivat ausgewählt ist unter:
- 2-Methyl-dibenzoylmethan,
- 4-Methyl-dibenzoylmethan,
- 4-Isopropyl-dibenzoylmethan,
- 4-tert.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4,4'-Dimethoxy-dibenzoylmethan,
- 4*-tert.*-Butyl-4'-methoxy-dibenzoylmethan,
- 2-Methyl-5-isopropyl-4'-methoxy-dibenzoylmethan,
- 2-Methyl-5*-tert.*-butyl-4'-methoxy-dibenzoylmethan,
- 2,4-Dimethyl-4'-methoxy-dibenzoylmethan, und
- 2,6-Dimethyl-4-*tert.*-butyl-4'-methoxy-dibenzoylmethan.

8. Verfahren nach Anspruch 7, **dadurch gekennzeichnet, daß** das Dibenzoylmethanderivat das 4-*tert*.-Butyl-4'-methoxy-dibenzoylmethan ist.

9. Verfahren nach Anspruch 7, **dadurch gekennzeichnet, daß** das Dibenzoylmethanderivat das 4-Isopropyl-dibenzoylmethan ist.

10. Verfahren nach einem der Ansprüche 1 bis 4, **dadurch gekennzeichnet, daß** das oder die unlöslichen organischen UV-Filter in mikronisierter Form unter den organischen UV-Filtern vom Oxanilid-Typ, Triazin-Typ, Triazol-Typ, Vinylamid-Typ und Cinnamid-Typ ausgewählt sind.

11. Verfahren nach Anspruch 10, wobei die UV-Filter vom Oxanilid-Typ der folgenden Formel entsprechen: 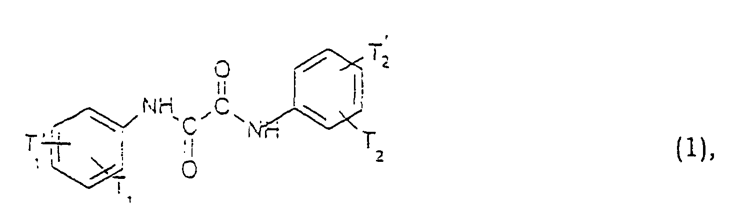 worin T₁, T'₁, T₂ und T'₂, die identisch oder voneinander verschieden sind, C₁₋₈-Alkyl oder C₁₋₈-Alkoxy bedeuten.

12. Verfahren nach Anspruch 10, wobei die Filter vom Triazin-Typ unter den unlöslichen s-Triazinderivaten ausgewählt sind, die Benzalmalonat- und/oder Phenylcyanoacrylatgruppen enthalten.

13. Verfahren nach Anspruch 12, wobei die Filter vom s-Triazin-Typ unter den folgenden Verbindungen ausgewählt sind:
- 2,4,6-Tris(diethyl-4'-amino-benzalmalonat)-s-triazin,
- 2,4,6-Tris(diisopropyl-4'-amino-benzalmalonat)-s-triazin,
- 2,4,6-Tris(dimethyl-4'-amino-benzalmalonat)-s-triazin,
- 2,4,6-Tris(ethyl-α-cyano-4-aminocinnamat)-s-triazin.

14. Verfahren nach Anspruch 10, wobei die unlöslichen Filter vom Triazin-Typ ausgewählt sind unter: 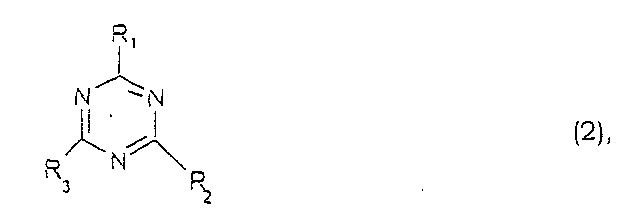 worin R₁, R₂ und R₃ unabhängig voneinander Phenyl, Phenoxy oder Pyrrolo bedeuten, wobei die Phenyl-, Phenoxy- oder Pyrrologruppen gegebenenfalls einen, zwei oder drei Substituenten tragen, die unter OH, C₁₋₁₈-Alkyl, C₁₋₁₈-Alkoxy, C₁₋₁₈-Carboxyalkyl, C₅₋₈-Cycloalkyl, Methylidencampher oder einer Gruppe -(CH=CH)ₙ(CO)-OR₄ ausgewählt sind, wobei R₄ entweder C₁₋₁₈-Alkyl oder Cinnamyl bedeutet und n 0 oder 1 ist.

15. Verfahren nach Anspruch 10, wobei die UV-Filter vom Triazin-Typ unter den unlöslichen s-Triazinderivaten ausgewählt sind, die Benzotriazol- und/oder Benzothiazolgruppen enthalten.

16. Verfahren nach Anspruch 15, wobei die Filter vom Triazin-Typ unter
- 2,4,6-Tris[(3'-benzotriazol-2-yl-2'-hydroxy-5'-methyl)phenylamino]-s-triazin und
- 2,4,6-Tris[(3'-benzotriazol-2-yl-2'-hydroxy-5'-tert.-octyl)phenylamino]-s-triazin ausgewählt sind.

17. Verfahren nach Anspruch 10, wobei die organischen UV-Filter vom Triazol-Typ der folgenden Formel (3) entsprechen: 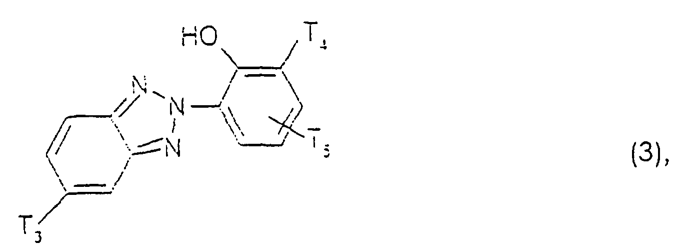 worin T₃ ein Wasserstoffatom oder eine C₁₋₁₈-Alkylgruppe bedeutet und T₄ und T₅, die identisch oder voneinander verschieden sind, eine gegebenenfalls mit Phenyl substituierte C₁₋₁₈-Alkylgruppe bedeuten.

18. Verfahren nach Anspruch 17, wobei die Verbindungen der Formel (3) unter den folgenden Verbindungen ausgewählt sind:

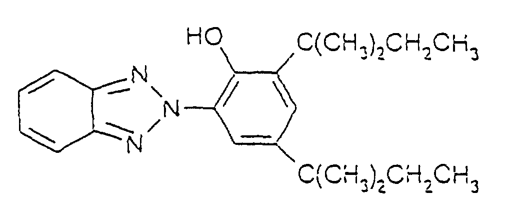

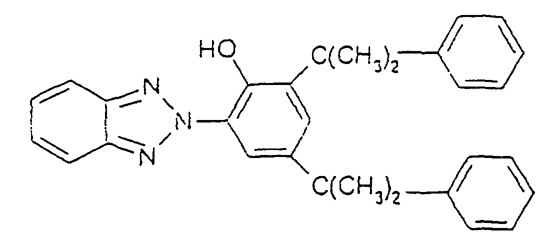

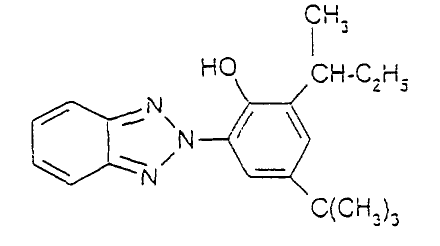

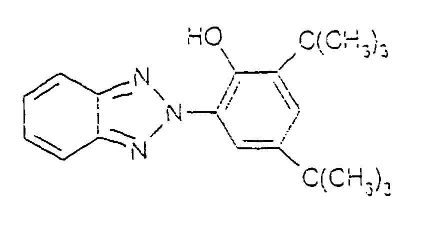

19. Verfahren nach Anspruch 10, wobei das unlösliche UV-Filter das [2,4'-Dihydroxy-3-(2H-benzotriazol-2-yl)-5-(1,1,3,3-tetramethylbutyl)-2'-n-octoxy-5'-benzoyl]-diphenylmethan der folgenden Struktur ist:

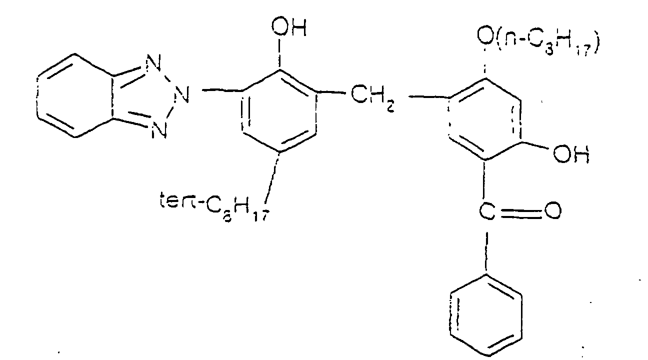

20. Verfahren nach Anspruch 10, wobei die organischen UV-Filter von Triazol-Typ unter den Methylen-bis(hydroxyphenyl-benzotriazol)-derivaten der folgenden Struktur ausgewählt sind: 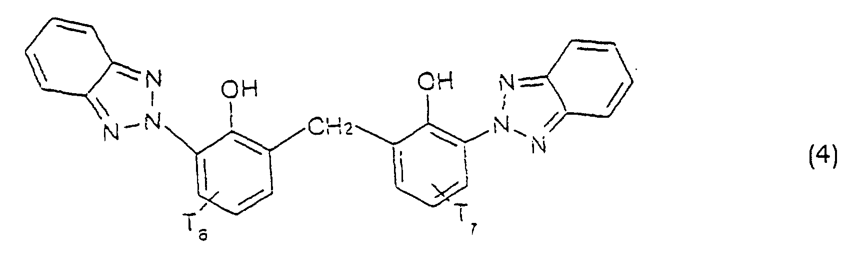 worin die Gruppen T₆ und T₇, die identisch oder voneinander verschieden sind, eine C₁₋₁₈-Alkylgruppe bedeuten, die mit einer oder mehreren Gruppen substituiert sein kann, die unter C₁₋₄-Alkyl, C₅₋₁₂-Cycloalkyl oder Aryl ausgewählt sind.

21. Verfahren nach Anspruch 20, wobei die Verbindung der Formel (4) unter den Verbindungen der folgenden Struktur ausgewählt ist:

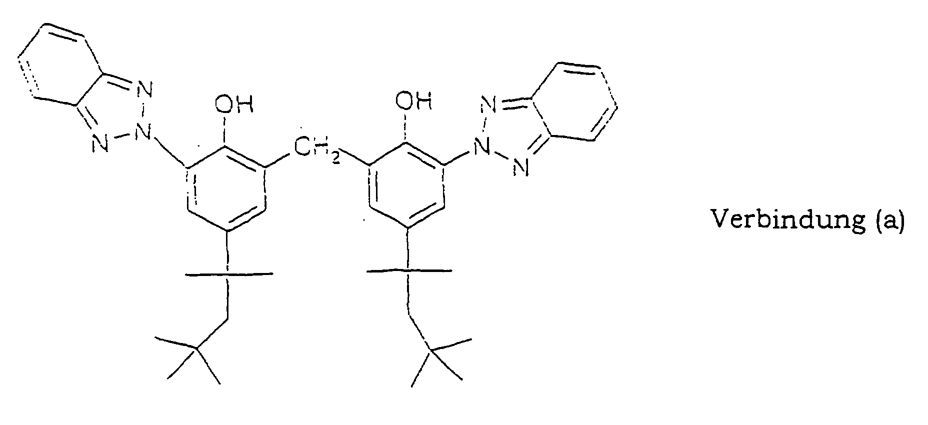

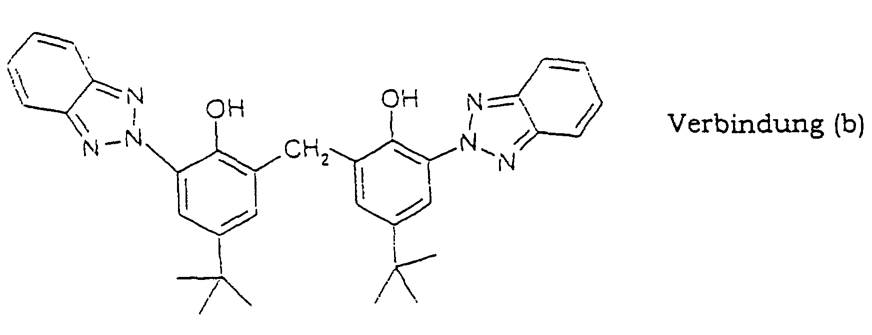

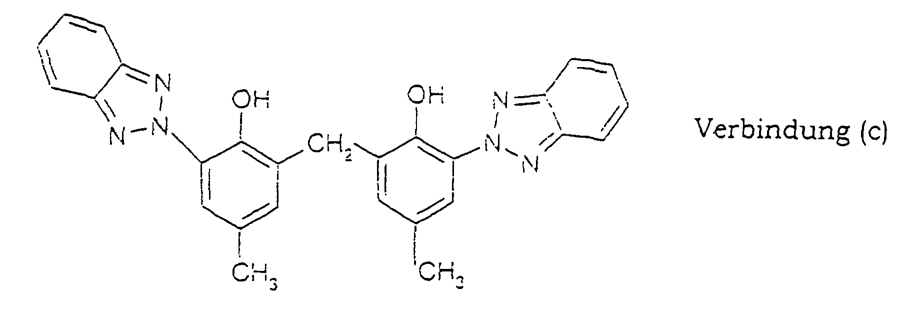

22. Verfahren nach Anspruch 20, wobei die organischen Filter vom Vinyl-amid-Typ der folgenden Formel entsprechen:
T₈-(Y)ᵣ-C(=O)-C(T₉)=C(T₁₀)-N(T₁₁)(T₁₂) (5)
worin T₈ eine C₁₋₁₈-Alkylgruppe und vorzugsweise eine C₁₋₅-Alkylgruppe oder eine Phenylgruppe bedeutet, die gegebenenfalls mit einer, zwei oder drei Gruppen substituiert ist, die unter OH, C₁₋₁₈-Alkyl, C₁₋₈-Alkoxy oder -C(=O)-OT₁₃ ausgewählt sind, wobei T₁₃ C₁₋₁₈-Alkyl bedeutet; T₉, T₁₀, T₁₁ und T₁₂, die identisch oder voneinander verschieden sind, bedeuten C₁₋₁₈-Alkyl und vorzugsweise C₁₋₅-Alkyl oder Wasserstoff; Y bedeutet N oder O und r 0 oder 1.

23. Verfahren nach Anspruch 22, wobei die Verbindungen der Formel (5) ausgewählt sind unter:
- 4-Octylamino-3-penten-2-on;
- Ethyl-3-octylamino-2-butenoat;
- 3-Octylamino-1-phenyl-2-buten-1-on; und
- 3-Dodecylamino-1-phenyl-2-buten-1-on.

24. Verfahren nach Anspruch 10, wobei die organischen UV-Filter vom Cinnamamid-Typ der folgenden Struktur entsprechen: 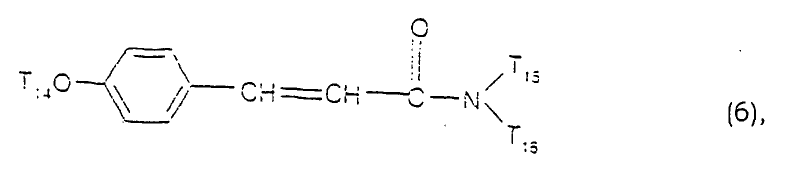 worin OT₁₄ eine Hydroxygruppe oder C₁₋₄-Alkoxy und vorzugsweise Methoxy oder Ethoxy bedeutet; T₁₅ bedeutet Wasserstoff oder C₁₋₄-Alkyl und vorzugsweise Methyl oder Ethyl; T₁₆ ist eine Gruppe -(CONH)ₛ-phenyl, wobei s 0 oder 1 ist und die Phenylgruppe mit einer, zwei oder drei Gruppen substituiert sind kann, die unter OH, C₁₋₁₈-Alkyl, C₁₋₈-Alkoxy oder -C(=O)-OT₁₇ ausgewählt sind, wobei T₁₇ eine C₁₋₁₈-Alkylgruppe und besonders bevorzugt Phenyl, 4-Methoxyphenyl oder Phenylaminocarbonyl ist.

25. Verfahren nach Anspruch 10, wobei das unlösliche UV-Filter eine Cinnamamid-Dimer ist.

26. Verfahren nach Anspruch 25, wobei das unlösliche UV-Filter die Verbindung der folgenden Struktur ist:

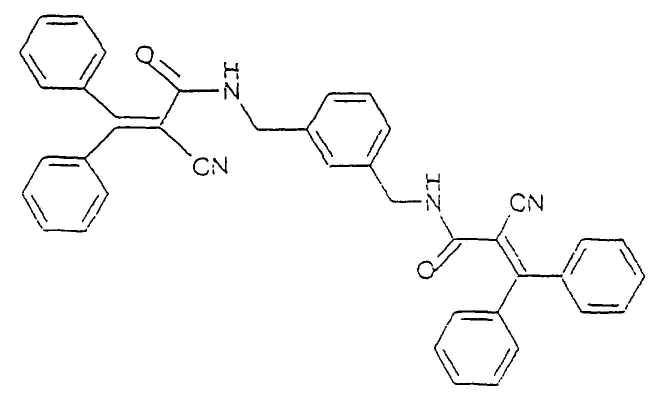

27. Verfahren nach einem der Ansprüche 1 bis 9, wobei die unlöslichen UV-Filter Salze von mehrwertigen Metallen und organischen UV-Filtern mit Sulfonsäure- oder Carboxygruppe sind.

28. Verfahren nach Anspruch 27, wobei die unlöslichen UV-Filter unter den Salzen von mehrwertigen Metallen und sulfonierten Benzylidencampherderivaten; Salzen von mehrwertigen Metallen und sulfonierten Benzimidazolderivaten; oder Salzen von mehrwertigen Metallen und Zimtsäurederivaten ausgewählt sind.

29. Verfahren nach einem der Ansprüche 1 bis 9, wobei die unlöslichen UV-Filter Komplexe von mehrwertigen Metallen oder Ammonium oder substituiertem Ammonium und organischen UV-A- und/oder UV-B-Filtem sind.

30. Verwendung eines unlöslichen organischen UV-Filters in mikronisierter Form nach einem der Ansprüche 1 bis 29 zur Herstellung von kosmetischen oder dermatologischen Zusammensetzungen, die mindestens ein Dibenzoylmethanderivat enthalten, um die Stabilität des Dibenzoylmethanderivats gegenüber UV-Strahlung zu verbessern.

## Claims

1. Process for improving the stability of at least one dibenzoylmethane derivative with respect to UV radiation, **characterized in that** it consists in combining the said dibenzoylmethane derivative with an effective amount of at least one insoluble organic UV screening agent in the micronized insoluble form with a mean particle size ranging from 0.01 to 2 µm, the said insoluble organic UV screening agent having a solubility in water at 25°C of less than 0.1% by weight and a solubility in liquid paraffin at 25°C of less than 1% by weight.

2. Process according to Claim 1, where the mean particle size varies from 0.02 to 1.5 µm.

3. Process according to Claim 2, where the mean particle size varies from 0.03 to 1.0 µm.

4. Process according to any one of Claims 1 to 4, **characterized in that** the insoluble UV screening agent or agents in the micronized form are capable of being obtained by a process for milling the insoluble organic screening agent in the form of coarse particles in the presence of a surfactant.

5. Process according to Claim 4, where the surfactant is chosen from alkylpolyglucosides with the structure CₙH₂ₙ₊₁O(C₆H₁₀O₅)ₓH, in which n is an integer from 8 to 16 and x is the mean degree of, polymerization of the (C₆H₁₀O₅) unit and varies from 1.4 to 1.6.

6. Process according to either one of Claims 4 and 5, **characterized in that** the surfactant is used at a concentration ranging from 1 to 50% by weight with respect to the insoluble organic UV screening agent in its micronized form.

7. Process according to any one of Claims 1 to 6, **characterized in that** the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

8. Process according to Claim 7, **characterized in that** the dibenzoylmethane derivative is 4-(tert-butyl)-4'-methoxydibenzoylmethane.

9. Process according to Claim 7, **characterized in that** the dibenzoylmethane derivative is 4-isopropyldibenzoylmethane.

10. Process according to any one of Claims 1 to 4, where the insoluble organic UV screening agent or agents in the micronized form are chosen from organic UV screening agents of the oxanilide type, of the triazine type, of the triazole type, of the vinyl amide type or of, the cinnamide type.

11. Process according to Claim 10, where the UV screening agents of the oxanilide type are of formula 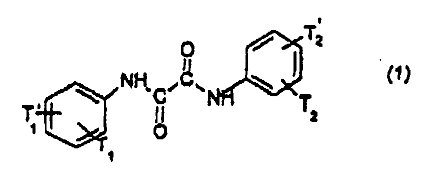 in which T₁, T'₁, T₂ and T'₂, which are identical or different, denote a C₁-C₈ alkyl radical or a C₁-C₈ alkoxy radical.

12. Process according to Claim 10, where the UV screening agents of the triazine type are chosen from insoluble s-triazine derivatives carrying benzalmalonate and/or phenylcyanoacrylate groups.

13. Process according to Claim 12, where the UV screening agents of the triazine type are chosen from the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

14. Process according to Claim 10, where the insoluble UV screening agents of the triazine type are chosen from 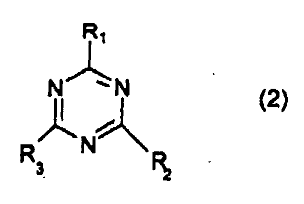 in which R₁, R₂ and R₃ are independently phenyl, phenoxy or pyrrolo, in which the phenyl, phenoxy or pyrrolo groups are optionally substituted by one, two or three substituents chosen from OH, C₁-C₁₈ alkyl or alkoxy, C₁-C₁₈ carboxyalkyl, C₅-C₈ cycloalkyl, a methylidenecamphor group or a -(CH=CH)ₙ(CO)-OR₄ group, with R₄ either C₁-C₁₈ alkyl or cinnamyl and n has the value 0 or 1.

15. Process according to Claim 10, where the UV screening agents of the triazine type are chosen from insoluble s-triazine derivatives carrying benzotriazole and/or benzothiazole groups.

16. Process according to Claim 15, where the UV screening agents of the triazine type are chosen from
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-tert-octyl)phenylamino]-s-triazine.

17. Process according to Claim 10, where the organic W screening agents of the triazole type correspond to the following formula (3): 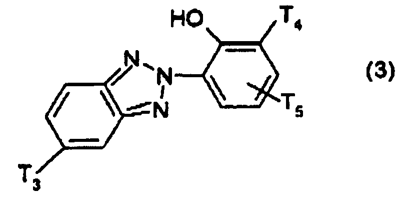 in which T₃ denotes a hydrogen atom or a C₁-C₁₈ alkyl radical, and T₄ and T₅, which are identical or different, denote a C₁-C₁₈ alkyl radical optionally substituted by a phenyl.

18. Process according to Claim 17, where the compound of formula (3) is chosen from the following compounds:

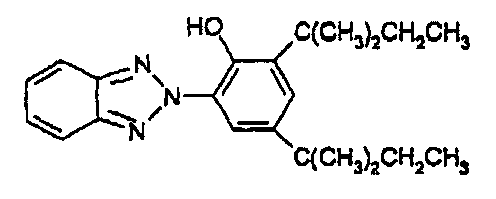

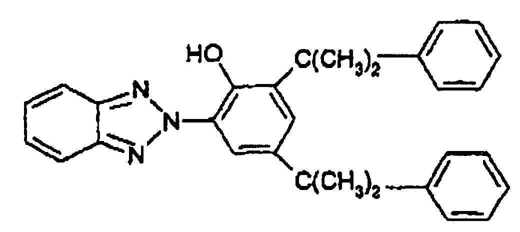

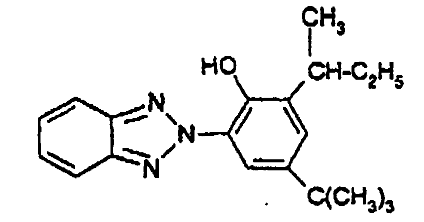

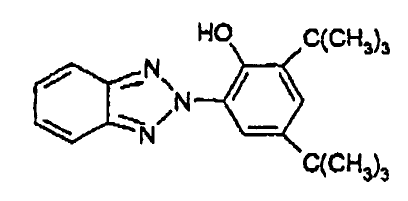

19. Process according to Claim 10, where the insoluble UV screening agent is [2,4'-dihydroxy-3-(2H-benzotriazol-2-yl)-5-(1,1,3,3-tetramethylbutyl)-2'-n-octoxy-5'-benzoyl]diphenylmethane with the structure:

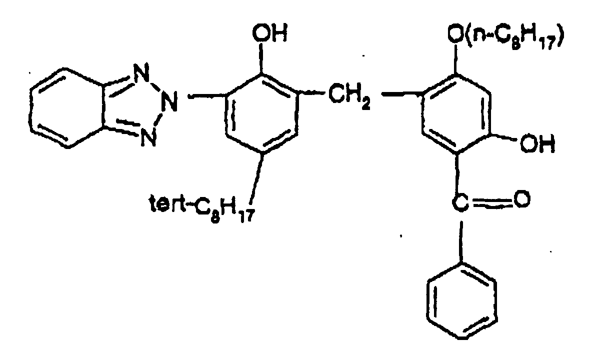

20. Process according to Claim 10, where the organic UV screening agents of the triazole type are chosen from methylenebis (hydroxyphenylbenzotriazole) derivatives with the following structure: 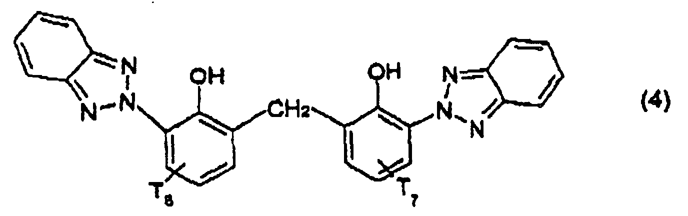 in which the T₆ and T₇ radicals, which are identical or different, denote a C₁-C₁₈ alkyl radical which can be substituted by one or more radicals chosen from C₁-C₄ alkyl, C₅-C₁₂ cycloalkyl or an aryl residue.

21. Process according to Claim 20, where the compound of formula (4) is chosen from the group consisting of the compounds with the following structure:

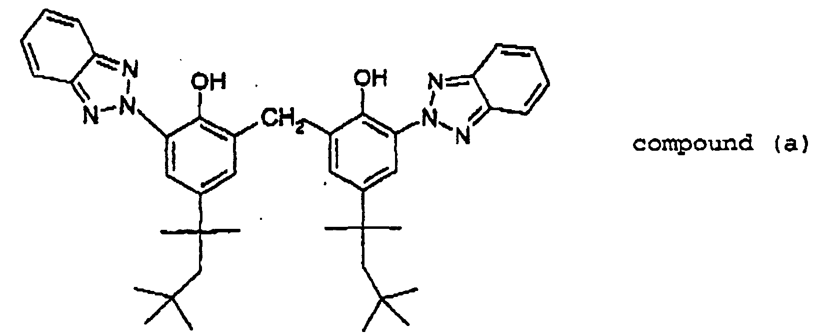

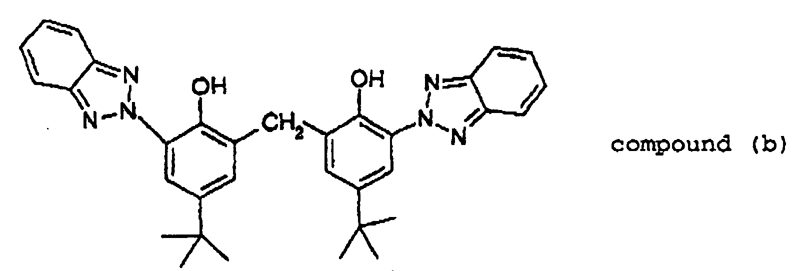

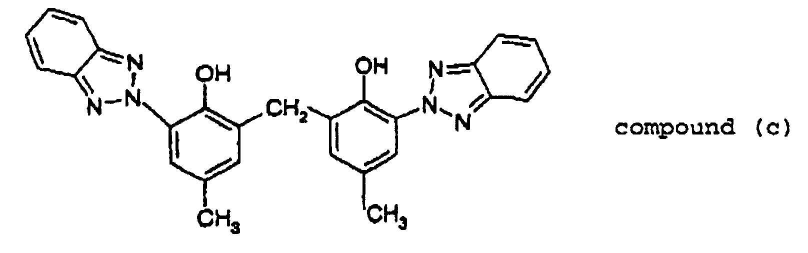

22. Process according to Claim 10, where the organic screening agents of the vinyl amide type correspond to the following formula:
T₈-(Y)r-C(=O)-C(T₉)=C(T₁₀) -N(T₁₁) (T₁₂) (5)
in which T₈ is a C₁-C₁₈, preferably C₁-C₅, alkyl radical or a phenyl group optionally substituted by one, two or three radicals chosen from OH, C₁-C₁₈ alkyl, C₁-C₈ alkoxy, or a -C(=O)-OT₁₃ group where T₁₃ is a C₁-C₁₈ alkyl; T₉, T₁₀, T₁₁ and T₁₂, which are identical or different, denote a C₁-C₁₈, preferably C₁-C₅, alkyl, radical or a hydrogen atom; Y is N or O and r has the value 0 or 1.

23. Process according to Claim 22, where the compounds of formula (5) are chosen from;
- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

24. Process according to Claim 10, where the organic screening agents of the cinnamamide type correspond to the following formula: 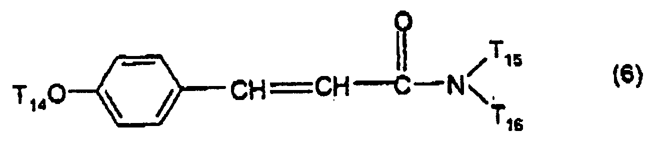 in which OT₁₄ is a hydroxyl or C₁-C₄ alkoxy, preferably methoxy or ethoxy, radical; T₁₅ is hydrogen or C₁-C₄ alkyl, preferably methyl or ethyl; T₁₆ is a -(CONH)s-phenyl group where s has the value 0 or 1 and the phenyl group can be substituted by one, two or three groups chosen from OH, C₁-C₁₈ alkyl, C₁-C₈ alkoxy, or a -C(=O)-OT₁₇ group where T₁₇ is a C₁-C₁₈ alkyl and more preferably T₁₇ is a phenyl, 4-methoxyphenyl or phenylaminocarbonyl group.

25. Process according to Claim 10, where the insoluble UV screening agent is a cinnamamide dimer.

26. Process according to Claim 25, where the insoluble UV screening agent is the compound with the structure:

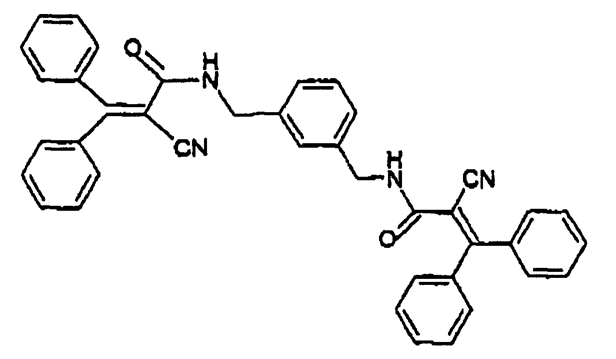

27. Process according to any one of Claims 1 to 9, where the insoluble UV screening agents are polyvalent metal salts of sulphonic or carboxylic organic UV screening agents.

28. Process according to Claim 27, where the insoluble UV screening agents are chosen from polyvalent metal salts of sulphonated benzylidenecamphor derivatives; polyvalent metal salts of sulphonated benzimidazole derivatives; or polyvalent metal salts of cinnamic acid derivatives.

29. Process according to any one of Claims 1 to 9. where the insoluble UV screening agents are polyvalent metal or ammonium or substituted ammonium complexes of organic UV-A and/or UV-B screening agents.

30. Use of an insoluble organic UV screening agent in the micronized form as defined according to any one of Claims 1 to 29 in the preparation of a cosmetic or dermatological composition comprising at least one dibenzoylmethane derivative, in order to improve the stability of the said dibenzoylmethane derivative with respect to UV radiation.
